# EUROPÄISCHE PATENTSCHRIFT

(11) **EP 1 537 892 B1**
(45) Veröffentlichungstag und Bekanntmachung des Hinweises auf die Patenterteilung: **03.01.2007**
(21) Anmeldenummer: 04028111.5
(22) Anmeldetag: 26.11.2004
(51) Int. Cl.: A61M 29/02

(54) **Ballonkatheter**
Balloon catheter
Cathèter à ballonet

(30) Priorität: 03.12.2003 DE 10356518
(43) Veröffentlichungstag der Anmeldung: 08.06.2005
(73) Patentinhaber: B. BRAUN MELSUNGEN AG, 34212 Melsungen (DE)
(72) Erfinder: Calisse, Jorge, Dr., 14199 Berlin (DE); Wöbken, Henning, 12305 Berlin (DE)
(74) Vertreter: von Kreisler Selting Werner

(56) Entgegenhaltungen:
- EP-A- 0 365 993
- WO-A-96/09848
- US-A- 4 323 071
- US-A- 4 960 410
- US-A- 4 964 853
- US-A- 5 826 588
- US-A- 6 165 152

## Beschreibung

Die Erfindung betrifft einen Ballonkatheter und insbesondere einen PTCA-Katheter zur Dilatation von Stenosen im Gefäßsystem.

Für die Behandlung von Blutgefäßen des Vascularsystems werden PTCA-Katheter eingesetzt (Percutaneous Transluminal Coronary Angioplasty). Hierbei handelt es sich um Ballonkatheter, die über einen Führungsdraht durch die Krümmungen des Vascularsystems an den Zielort geschoben werden.

Die distale Spitze eines PTCA-Ballonkatheters muss eine hohe Kompressionsstabilität haben, damit sie enge Stenosen passieren kann. Trotz der Kompressionsstabilität muss die Katheterspitze flexibel sein, damit sich der gesamte Katheter in das gewundene Gefäßsystem einbringen lässt. Bei herkömmlichen PTCA-Kathetern ist der distale (patientenseitige) Schlauchabschnitt, also der Schlauchabschnitt jenseits des Ballons, aus Abschnitten verschiedener Materialien mit unterschiedlichen mechanischen Eigenschaften gefertigt. Einzelne Schlauchstücke werden hintereinandergesetzt und durch Schweißen verbunden. Dies ist mit einem hohen Fertigungsaufwand und entsprechend hohen Kosten verbunden.

Ein Ballonkatheter, von dem der Oberbegriff des Patentanspruchs 1 ausgeht, ist beschrieben in US 4,960,410 A. Bei diesem Ballonkatheter ist der Innenschlauch mit einer spiralförmigen Aussparung versehen, die sich vom distalen Ende durch den Ballon hindurch erstreckt. Die spiralförmige Aussparung dient der Erhöhung der Flexibilität des Innenschlauchs. Der Bereich, der die Aussparung enthält, ist mit einem zusätzlichen Mantel umhüllt, um die Außenfläche des Innenschlauchs zu glätten. Dieser Mantel ist im Bereich des distalen Endes des Innenschlauchs mit einer Manschette überdeckt, welche aus der Haut des Ballons besteht.

US 6,165,152 A beschreibt einen Ballonkatheter, bei dem der Innenschlauch in einem distalen Schlauchabschnitt mit Aussparungen versehen ist, wodurch die Flexibilität dieses Schlauchabschnitts erhöht wird. Die Aussparungen erstrecken sich nur partiell durch die Schlauchwand, d.h. sie sind nicht durchgehend. Die mit einem Laserstrahl erzeugten Aussparungen können aus einer schraubenförmigen Linie oder diskreten Sacklöchern bestehen. Sie bewirken eine Strukturierung bzw. Aufrauhung der Außenfläche des distalen Schlauchabschnitts.

WO 96/09848 beschreibt einen Ballonkatheter mit Innenschlauch und Außenschlauch. Der Ballon ist an dem Innenschlauch durch Wärmeschrumpfen fixiert. Die Ballonhaut überragt das distale Ende des Innenschlauchs und bildet eine flexible Katheterspitze.

Der vorliegenden Erfindung liegt die Aufgabe zugrunde, einen Ballonkatheter zu schaffen, dessen distaler Schlauchabschnitt zur Erzielung einer gewünschten Biegsamkeit zwar bearbeitet ist, jedoch ohne dass die Glätte der Oberfläche nachteilig verändert wäre.

Der erfindungsgemäße Ballonkatheter weist die Merkmale des Patentanspruchs 1 auf. Hiernach ist vorgesehen, dass die Aussparungen aus durch die Schlauchwand durchgehenden oder nicht durchgehenden diskreten Löchern bestehen und dass die Manschette die gesamte, mit Aussparungen versehene Länge des Innenschlauchs, eng umschließt. Die Ballonhaut erstreckt sich also über den gesamten durch nachträgliche Bearbeitung strukturierten Oberflächenbereich des Innenschlauchs bis in die unmittelbare Nähe der Katheterspitze.

Da die Aussparungen aus diskreten Löchern bestehen, bewirkt die aufgeschweißte Manschette eine bessere Lastverteilung, wodurch örtliche Spannungsspitzen verhindert werden. Außerdem wird die Knickgefahr verringert.

Während bei dem US-Patent 6,165,152 der strukturierte Bereich durch die distale Manschette des Ballons hindurchgeht und dadurch zwangsläufig auf einem Teil seiner Länge ohne besondere Absicht bedeckt ist, wird erfindungsgemäß die Ballonhaut dazu benutzt, die zuvor strukturierte Oberfläche zu bedecken und den durch das Entfernen von Material hervorgerufene Strukturänderung der Oberfläche zu heilen. Somit wird die Gefahr einer Verletzung oder des Ablösens von Gefäßablagerungen aufgrund der Aussparungen und bei Biegung des distalen Schlauchabschnitts verringert. Die Erfindung geht von dem Gedanken aus, dass die Haut des Ballons eine geringe Dicke hat und damit eine ideale Umhüllung zum glättenden engen Umspannen des distalen Schlauchabschnitts des Innenschlauches bildet.

Gemäß einer bevorzugten Ausgestaltung der Erfindung ist die distale Manschette auf die mit den Aussparungen versehene Länge aufgeschweißt. Beim Schweißen der distalen Manschette auf den durch die Aussparungen geschwächten distalen Schlauchabschnitt ergibt sich eine glatte Außenfläche, die vorteilhaft beim Durchdringen einer Stenose ist. Durch die angeschweißte umhüllende Ballonschicht wird ferner die Flexibilität des Schlauchabschnitts vergleichmäßigt.

Die distale Manschette kann aus einem Schrumpfschlauch bestehen. Ein Schrumpfschlauch nimmt durch Erwärmung wieder seine Ursprungsform an. Im vorliegenden Fall schrumpft er an die Außenfläche des Innenschlauchs an, wobei zusätzlich eine Schweißverbindung durchgeführt werden kann.

Die Herstellung der Aussparungen erfolgt vorzugsweise mit einem UV-Laser oder Excimerlaser oder mit mechanischen Verfahren. Mit Laserbearbeitung kann auch eine kegelförmige Katheterspitze geformt werden. Bevorzugt wird hierbei ein CO₂-Laser. Hierbei wird das Schlauchmaterial nicht abgebaut, sondern verschmolzen und verschoben.

Im Folgenden werden unter Bezugnahme auf die Zeichnungen Ausführungsbeispiele der Erfindung näher erläutert.

Es zeigen:
- Fig. 1: eine Teildarstellung des Ballonkatheters, teilweise im Längsschnitt,
- Fig. 2: eine Seitenansicht des distalen Schlauchabschnitts des Innenschlauchs, und
- Fig. 3: ein zweites Ausführungsbeispiel eines Ballonkatheters nach der Erfindung.

Der Ballonkatheter weist einen langgestreckten Außenschlauch 10 und einen in dem Außenschlauch verlaufenden Innenschlauch 11 auf. Am distalen Ende des Außenschlauchs 10 ist ein Ballon 12 befestigt, in den das Lumen 13 des Außenschlauchs mündet. Durch Einpressen einer Druckflüssigkeit in das Lumen 13 wird der Ballon 12 aufgeweitet, um eine Dilatation einer Stenose durchzuführen. Die Schläuche bestehen aus Polyamid oder aus geeigneten Materialmischungen.

Der Innenschlauch 11 erstreckt sich durch den Ballon 12 hindurch. Der distale Schlauchabschnitt 14 endet in einer konisch verjüngten Katheterspitze 15. Durch das Lumen 16 des Innenschlauchs 11 kann ein (nicht dargestellter) Führungsdraht vorgeschoben werden, der aus der distalen Katheterspitze 15 heraus vorsteht.

Der Ballon 12 ist mit einer proximalen Manschette 17 auf den Außenschlauch 10 aufgeschweißt. Am distalen Ende ist der Ballon 12 mit einer distalen Manschette 18 versehen, die an den Innenschlauch 11 angeschweißt ist. Die Manschetten sind vorzugsweise integraler Bestandteil des Ballons. Sie können alternativ auch angeschweißt sein bzw. aus mehreren Teilen bestehen.

Der distale Schlauchabschnitt 14 des Innenschlauchs 11 ist mit Aussparungen 20 versehen, um die Flexibilität des Schlauchabschnitts zu erhöhen. Der Schlauchabschnitt 14 ist einstückiger Bestandteil des Innenschlauchs 11. Die Aussparungen 20 sind bei dem vorliegenden Ausführungsbeispiel Löcher, die mit einem Laserstrahl eingebrannt sind und die Katheterwand durchdringen. Alternativ besteht die Möglichkeit, die Aussparungen als nicht durchgehende Schwächungsstellen auszubilden. Die bevorzugte Form der Aussparungen ist rund. Die Aussparungen können aber jede beliebige Form haben, beispielsweise Ellipsenform mit in Schlauchlängsrichtung verlaufender Hauptachse, oder Rechteckform. Die Herstellung von durchgehenden Aussparungen in Form von Löchern ist einfacher und führt nicht zu unbeabsichtigten Undichtigkeiten, weil der gesamte Bereich der Aussparungen mit der Haut 21 des Ballons 12 eng anliegend umhüllt ist. Dies bedeutet, dass die Manschette 18 sich über die gesamte Länge des Aussparungsbereichs erstreckt und sämtliche Aussparungen bedeckt. Dadurch wird die Außenfläche des distalen Schlauchabschnitts 14 geglättet.

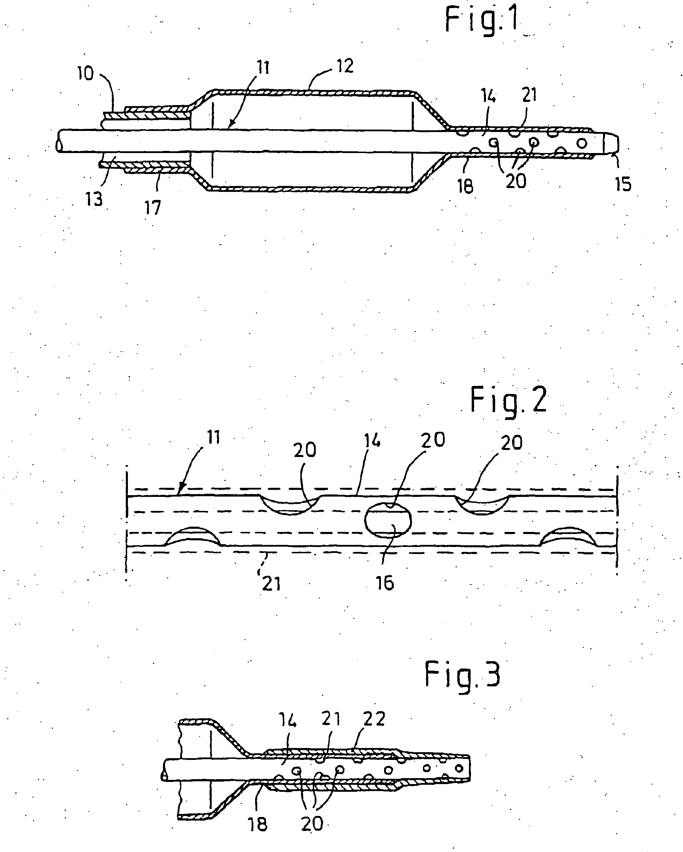

Figur 2 zeigt eine Seitenansicht des mit den Aussparungen 20 versehenen distalen Schlauchabschnitts 14 des Innenschlauchs 11, wobei die Ballonhaut 21, die die Aussparungen bedeckt, gestrichelt dargestellt ist. Die Ballonhaut 21 ist vollflächig mit dem Schlauchabschnitt 14 verbunden, insbesondere durch Schweißung. Sie bewirkt daher einen Spannungsausgleich und eine Überbrückung der Aussparungen 20.

Figur 3 zeigt eine modifizierte Ausführungsform des Spitzenbereichs des Ballonkatheters. Der Bereich des Innenschlauchs 11, an dem die Aussparungen 20 vorgesehen sind, ist auch hier mit der Manschette 18 des Ballons 12 bedeckt. Auf der Manschette 18 sitzt ein Schrumpfschlauch 22, der sich bis zum distalen Ende des Innenschlauchs erstreckt und ggf. darüber hinausragt. Wird der Schrumpfschlauch 22 erwärmt, beispielsweise durch Laserbestrahlung, so verringert sich sein Durchmesser. Durch den Druck, den der Schrumpfschlauch 22 ausübt und durch die Wärme der Laserbestrahlung wird die Manschette 18 an den Innenschlauch 11 angeschweißt und gleichzeitig geformt. Bei der Formung der Katheterspitze erfolgt eine Steuerung des Laserprozesses. Der Schrumpfschlauch 22 verbleibt bei dieser Ausführungsform nach dem Schrumpfvorgang an dem Katheter.

Bei einer weiteren nicht dargestellten Ausführungsform des Spitzenbereichs des Ballonkatheters wird der bereits beschriebene Schrumpfschlauch nach dem Schrumpfvorgang vom Katheter entfernt. Der Bereich des Innenschlauchs, an dem die Aussparungen vorgesehen sind, wird bei dieser Ausführungsform vollständig von der Manschette des Ballons bedeckt.

## Patentansprüche

1. Ballonkatheter mit einem Außenschlauch (10), der mit einem Ballon (12) verbunden ist, einem Innenschlauch (11), der durch den Ballon (12) hindurchgeht und einen distalen Schlauchabschnitt (14) aufweist, wobei der distale Schlauchabschnitt (14) Aussparungen (20) zur Erhöhung der Flexibilität aufweist und die Haut des Ballons (12) eine distale Manschette (18) bildet, die zur Erzielung einer geglätteten Außenfläche die mit den Aussparungen (20) versehene Länge des Innenschlauchs (11) bedeckt,
**dadurch gekennzeichnet,**
**dass** die Aussparungen (20) aus durch die Schlauchwand durchgehenden oder nicht durchgehenden diskreten Löchern bestehen und dass die Manschette (18) die gesamte mit Aussparungen versehene Länge des Innenschlauchs eng umschließt.

2. Ballonkatheter nach Anspruch 1, **dadurch gekennzeichnet, dass** die Manschette (18) auf die mit den Aussparungen (20) versehene Länge des Innenschlauchs (11) aufgeschweißt ist.

3. Ballonkatheter nach Anspruch 1 oder 2, **dadurch gekennzeichnet, dass** die distale Manschette (18) aus einem Schrumpfschlauch besteht.

4. Ballonkatheter nach einem der Ansprüche 1 - 3, **dadurch gekennzeichnet, dass** die distale Manschette (18) von einem Schrumpfschlauch (22) umgeben ist, der sich über das distale Ende der Manschette hinaus erstreckt und das daraus hervorstehende Ende des Innenschlauchs (11) umschließt.

## Claims

1. A balloon catheter comprising an outer tube (10) connected with a balloon (12), an inner tube (11) extending through said balloon (12) and having a distal tube section (14), wherein said distal tube section (14) is provided with recesses (20) for increasing the flexibility, and the skin of the balloon (12) forms a distal sleeve (18) covering the length provided with the recesses (20) of the inner tube (11) for presenting a smoothed outer surface,
**characterized in that**
the recesses (20) are formed by discrete holes penetrating or not penetrating the tube wall, and the sleeve (18) tightly embraces the overall length provided with recesses of the inner tube.

2. The balloon catheter according to claim 1, **characterized in that** the sleeve (18) is welded onto the length provided with the recesses (20) of the inner tube (11).

3. The balloon catheter according to claim 1 or 2, **characterized in that** the distal sleeve (18) is composed of a shrink tube.

4. The balloon catheter according to one of claims 1-3, **characterized in that** the distal sleeve (18) is surrounded by a shrink tube (22) extending beyond the distal end of the sleeve and embracing the end of the inner hose (11) projecting therefrom.

## Revendications

1. Cathéter à ballonnet comportant un tuyau extérieur (10) relié à un ballonnet (12), un tuyau intérieur (11) traversant le ballonnet (12) et présentant une section de tuyau distale (14), la section de tuyau distale (14) présentant des évidements (20) permettant d'augmenter la flexibilité, et la peau du ballonnet (12) formant un manchon distal (18) qui recouvre la longueur du tuyau interne (11) pourvu d'évidements (20) de manière à produire une surface extérieure lissée,
**caractérisé en ce que**
les évidements (20) consistent en des trous discrets traversant ou non la paroi du tuyau et **en ce que** le manchon (18) enveloppe étroitement la totalité de la longueur du tuyau intérieur pourvu d'évidements.

2. Cathéter à ballonnet selon la revendication 1, **caractérisé en ce que** le manchon (18) adhère par soudure sur la longueur du tuyau intérieur (11) pourvue des évidements (20).

3. Cathéter à ballonnet selon la revendication 1 ou 2, **caractérisé en ce que** le manchon distal (18) consiste en un tuyau thermorétractable.

4. Cathéter à ballonnet selon l'une des revendications 1 à 3, **caractérisé en ce que** le manchon distal (18) est entouré par un tuyau thermorétractable (22) qui s'étend sur l'extrémité distale du manchon et gaine l'extrémité du tuyau interne (11) qui en fait saillie.
